Europäisches Patentamt

European Patent Office

Office européen des brevets

(19)

(11) EP 0 838 676 A1

## (12) DEMANDE DE BREVET EUROPEEN

(43) Date de publication:
29.04.1998 Bulletin 1998/18

(51) Int Cl.⁶: $G01N\ 21/35$, $G01N\ 33/28$

(21) Numéro de dépôt: 97402492.9

(22) Date de dépôt: 21.10.1997

(84) Etats contractants désignés:
AT BE CH DE DK ES FI FR GB GR IE IT LI LU MC
NL PT SE

(30) Priorité: 23.10.1996 FR 9612918

(71) Demandeur: ELF ANTAR FRANCE
92400 Courbevoie (FR)

(72) Inventeur: Saby, Claude-Alain
69500 Lyon (FR)

(74) Mandataire: Timoney, Ian Charles Craig
Elf Exploration Production
Département Propriété Industrielle
Tour Elf
EP/T/RD/DPI - Bureau 34 G 47
92078 Paris La Défense Cedex (FR)

(54) Procédé de suivi et de surveillance d'une unité de fabrication et/ou d'un spectrometre proche infrarouge au moyen d'au moins un indicateur

(57) La présente invention concerne un procédé de suivi et de surveillance du fonctionnement d'une unité de fabrication d'un produit et/ou d'un spectromètre proche infrarouge alimenté par ledit produit.

Ce procédé consiste, à calculer au moins un indicateur de bon fonctionnement de l'unité de fabrication et/ou du spectromètre proche infrarouge, au moyen d'une expression mathématique combinant les valeurs de spectres de travail et celles de spectres recomposés correspondants et à suivre l'évolution de ces indicateurs. Ces spectres étant obtenus par transformations de spectres issus du spectromètre et enregistrés périodiquement.

Elle trouve son application dans les industries chimiques, pétrolières, pharmaceutiques, cosmétologiques et agro-alimentaires.

## Description

## DOMAINE TECHNIQUE

La présente invention concerne un procédé de suivi et de surveillance en ligne d'une unité de fabrication à l'aide des spectres issus d'un spectromètre proche infrarouge à laquelle il est raccordé. Le procédé de l'invention permet également le suivi et la surveillance du spectromètre lui-même.

Elle trouve son application dans les industries chimiques, pétrolières, pharmaceutiques, cosmétologiques et agro-alimentaires.

## ETAT DE LA TECHNIQUE ANTERIEURE

Le spectromètre proche infrarouge est généralement utilisé pour déterminer les caractéristiques physiques ou chimiques d'un échantillon de produit à analyser prélevé sur une unité de fabrication. Il est aussi utilisé pour suivre et surveiller le bon fonctionnement de l'unité de fabrication à laquelle il est raccordé ainsi que son propre bon fonctionnement. Pour ces utilisations une calibration du spectromètre est nécessaire.

Cette calibration consiste à établir un modèle qui représente la relation mathématique entre une caractéristique du produit à analyser et le spectre délivré par le spectromètre. Le modèle est élaboré par la mise en oeuvre de techniques statistiques multivariées telles que l'analyse en composantes principales, la régression en composantes principales, la régression selon les moindres carrés ou les réseaux de neurones.

Des méthodes connues de suivi et de surveillance d'unités de fabrication ou de spectromètres proche infrarouge consistent à sélectionner soit certains éléments du spectre, soit des variables issues des calculs de la modélisation, puis à suivre au moyen de cartes de contrôle ces éléments et/ou ces variables.

Une de ces méthodes connues est décrite dans la demande de brevet français n° 95 12087 déposée le 16 octobre 1995. Cette méthode de suivi du fonctionnement d'un instrument esclave et d'une unité de fabrication à laquelle il est raccordé, comprend une opération de calibration multivariée d'un analyseur maître, des opérations périodiques de standardisation des signaux délivrés par l'analyseur esclave alimenté par des produits de standardisation et une étape de transfert de calibration au cours de laquelle sont calculés des paramètres associés à un algorithme de transfert de calibration. Elle consiste en outre à choisir, d'une part, au moins un desdits paramètres ou une combinaison mathématique d'au moins deux d'entre eux comme indicateur de suivi et de contrôle du fonctionnement de l'analyseur esclave et d'autre part une méthode de suivi et de contrôle. A l'issue de chaque opération périodique de standardisation on surveille l'évolution dans le temps de la valeur de l'indicateur de suivi et de contrôle en appliquant la méthode de suivi et de contrôle, puis on vérifie le bon fonctionnement de l'analyseur esclave et celui de l'unité de fabrication à laquelle il est raccordé en identifiant les causes de l'évolution de la valeur de l'indicateur à partir, des résultats obtenus par l'application de la méthode de suivi et de contrôle et d'un diagramme causes / effets. Selon une caractéristique particulière de cette méthode l'analyseur esclave et l'analyseur maître sont le même analyseur utilisé à des périodes de temps différentes.

Cette méthode est particulièrement bien adaptée au suivi des spectromètres proche infrarouge. En revanche, elle ne permet pas toujours de distinguer les changements de production des dysfonctionnements de l'unité à laquelle un spectromètre est raccordé. Elle présente aussi l'inconvénient de nécessiter des produits de caractéristiques connues pour réaliser les opérations périodiques de standardisation.

## EXPOSE DE L'INVENTION

La présente invention a justement pour objet de remédier à ces inconvénients, et notamment de fournir un procédé de suivi et/ou de surveillance du fonctionnement d'un spectromètre proche infrarouge et de l'unité de fabrication à laquelle il est raccordé.

Grâce à ce procédé, il est possible de mettre en évidence des perturbations, des dérives, des anomalies de fonctionnement du spectromètre et de la chaîne de mesure associée ainsi que de l'unité de fabrication à laquelle il est raccordé, d'identifier les causes de ces dysfonctionnements et de prendre des dispositions adaptées à chaque situation : par exemple déclarer le résultat de l'analyse invalide et prévenir l'opérateur qui exploite l'unité à laquelle le spectromètre est raccordé et lui fournir des éléments pour prendre ses décisions.

Ce procédé trouve son application dans les laboratoires d'analyses et les unités de fabrication des industries chimiques, pétrolières, pharmaceutiques, cosmétologiques et agro-alimentaires.

A cette fin la présente invention propose un procédé de suivi et de surveillance au moyen d'au moins un indicateur, du fonctionnement d'une unité de fabrication d'un produit et/ou d'un spectromètre proche infrarouge alimenté par ledit produit, ledit spectromètre délivrant des spectres constitués par des séries de valeurs d'absorbance pour différentes valeurs de longueurs d'ondes, consistant à exécuter les étapes suivantes :

- enregistrer périodiquement sous forme de données numériques les spectres issus du spectromètre proche infrarouge,
- transformer mathématiquement les données numériques de chaque spectre enregistré, pour obtenir des spectres transformés,
- constituer une suite de spectres de travail à partir des valeurs précédemment obtenues, en sélectionnant dans chaque spectre transformé, un ensemble de longueurs d'ondes consécutives caractéristiques du produit fabriqué,

caractérisé en ce qu'il consiste à exécuter en plus les étapes suivantes :

- calculer des coefficients par décomposition en série de chaque spectre de travail,
- choisir un nombre n limité de coefficients en effectuant une opération de sélection,
- déterminer un spectre recomposé par recomposition de chaque spectre de travail à partir des coefficients choisis,
- calculer au moins un indicateur de bon fonctionnement de l'unité de fabrication et/ou du spectromètre proche infrarouge, au moyen d'une expression mathématique combinant les valeurs de chaque spectre de travail et celles du spectre recomposé correspondant,
- suivre dans le temps l'évolution de l'indicateur de bon fonctionnement.

Selon une autre caractéristique du procédé de l'invention la décomposition des spectres de travail est effectuée au moyen d'une décomposition en série choisie parmi les décompositions en série de Fourier, d'Hadamard et en série d'ondelettes.

Selon une autre caractéristique du procédé de l'invention, la suite de spectres de travail comportant un premier spectre, l'opération de sélection consiste à déterminer le nombre n de coefficients en exécutant les étapes suivantes :

- choisir dans le premier spectre de travail un nombre l de longueurs d'ondes consécutives,
- calculer des coefficients par décomposition en série du premier spectre,
- choisir les p premiers coefficients,
- déterminer un premier spectre recomposé par recomposition du premier spectre de travail à partir des p premiers coefficients,
- calculer un paramètre STDm par la formule :

$$STDm = [\Sigma_i(A_i-A'_i)^2/l]^{\frac{1}{2}}$$

dans laquelle :

- $A_i$ représente la valeur en absorbance transformée mathématiquement pour la longueur d'onde i du premier spectre de travail,
- $A'_i$ représente la valeur en absorbance transformée mathématiquement pour la longueur d'onde i du premier spectre de travail recomposé,
- i varie de 1 à l,
- l représente le nombre de longueurs d'ondes choisi
- réitérer le calcul du paramètre STDm avec les p+q premiers coefficients, q allant de 1 à l/4,
- comparer les valeurs STDm ainsi obtenues avec une valeur de seuil STDs,
- retenir le nombre n de coefficients correspondant à la valeur STDm immédiatement supérieure à la valeur STDs.

Selon une autre caractéristique du procédé de l'invention le spectromètre proche infrarouge ayant une répétabilité connue, la valeur de seuil STDs est choisie égale à l'écart type de ladite répétabilité.

Selon une autre caractéristique du procédé de l'invention, l'expression mathématique combinant les valeurs du spectre transformé et celles du spectre recomposé est la suivante :

$$STD = [\Sigma_j(A_j-A'_j)^2/l]^{\frac{1}{2}}$$

dans laquelle :

- STD représente l'indicateur de bon fonctionnement,
- Aj représente la valeur en absorbance transformée mathématiquement pour la longueur d'onde j de chaque spectre de travail,
- A'j représente la valeur en absorbance transformée mathématiquement pour la longueur d'onde j de chaque spectre de travail recomposé avec le nombre n de coefficients retenu,
- j varie de 1 à l
- l représente le nombre de longueurs d'ondes choisi
Selon une autre caractéristique du procédé de l'invention pour suivre l'évolution dans le temps de l'indicateur de bon fonctionnement, on utilise une carte de contrôle monovariée.

Selon une autre caractéristique du procédé de l'invention, on utilise un diagramme causes/effets pour suivre l'évolution dans le temps de l'indicateur de bon fonctionnement.

## BREVE DESCRIPTION DES DESSINS

L'invention sera mieux comprise à la lecture de la description qui suit, en référence aux dessins annexés dans lesquels :

- la figure 1 représente la dérivée première d'un spectre enregistré issu d'un spectromètre proche infrarouge,
- la figure 2 représente un spectre de travail,
- la figure 3 représente les valeurs d'un premier indicateur de bon fonctionnement d'une unité de fabrication aux instants d'analyse du produit fabriqué,
- la figure 4 représente les valeurs d'un deuxième indicateur de bon fonctionnement d'une unité de fabrication aux instants d'analyse du produit fabriqué.

## EXPOSE DETAILLE DE L'INVENTION

D'une manière générale, le procédé de l'invention permet de suivre et de surveiller le fonctionnement d'une unité de fabrication au moyen d'un spectromètre proche infrarouge. Il permet également le suivi et la surveillance du fonctionnement du spectromètre lui-même.

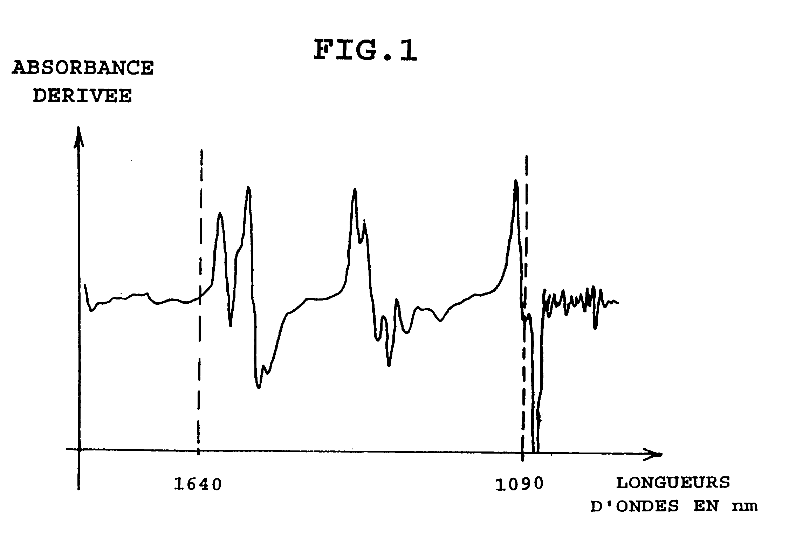

Le procédé de l'invention utilisé pour surveiller le bon fonctionnement d'une unité de production, par exemple la production d'essence dans une raffinerie de pétrole brut, consiste à enregistrer périodiquement dans un calculateur toutes les trois mn, sous forme de données numériques les résultats de la mesure d'absorbance de l'essence produite, au moyen d'un spectromètre proche infrarouge, dans la plage 907 nm à 1 793 nm. On obtient ainsi, avec un pas d'échantillonnage de 0,75 nm un ensemble de spectres constitués chacun par les 1 182 de valeurs d'absorbance mesurées sur cette plage de longueurs d'ondes. Chaque spectre est ensuite transformé par calcul de la dérivé première de l'absorbance par rapport à la longueur d'onde. Un exemple de ces spectres est représenté sur le diagramme de la figure 1 sur lequel on trouve en ordonnées les valeurs d'absorbance dérivées et en abscisses les longueurs d'ondes. Dans chaque spectre transformé on sélectionne deux plages de valeurs de longueurs d'ondes caractéristiques de l'essence produite, pour constituer deux spectres de travail :

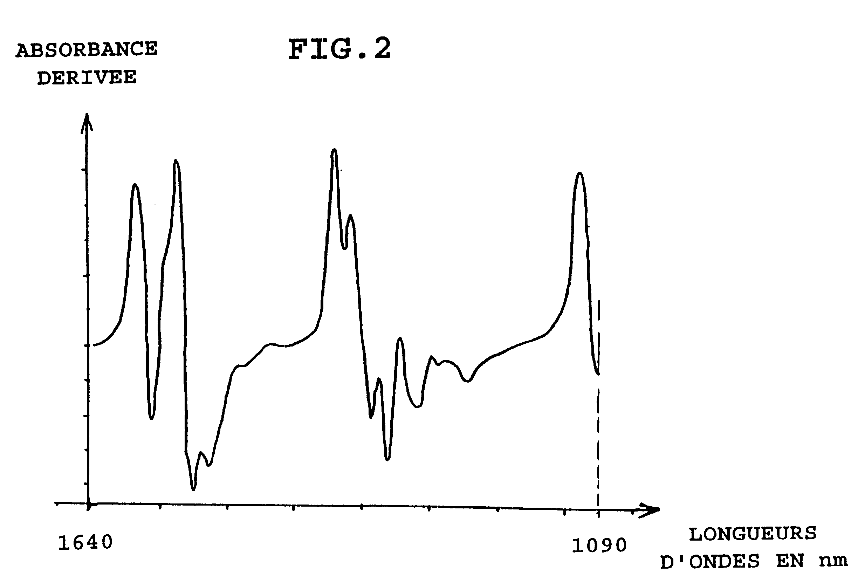

-   un premier spectre de travail représenté sur la figure 2 qui comprend les valeurs d'absorbance dérivées pour les longueurs d'ondes comprises entre 1 090 et 1 640 nm soit 735 couples de valeurs.
-   un deuxième spectre de travail qui comprend les valeurs d'absorbance dérivées pour les longueurs d'ondes comprises entre 1 090 et 1 540 nm.

Ces plages de longueurs d'ondes caractéristiques du produit fabriqué sont déterminées expérimentalement.

L'étape suivante du procédé de l'invention consiste à calculer des coefficients par décomposition en série de Fourier les spectres de travail en appliquant la formule suivante :

$$F(k) = (1/N)\Sigma_i\, y_i \exp(-j2i\pi k/N)$$

dans laquelle :

-   $y_i$ représente les valeurs d'absorbance d'un spectre de travail
-   F(k) représente les coefficients résultants de la décomposition en série de Fourier à la fréquence k/N,
-   N = 735 pour le premier spectre de travail
-   N = 600 pour le deuxième spectre de travail
-   i varie de 0 à N-1
-   k varie de 0 à N-1

On limite le nombre de coefficients k à une valeur n très inférieure à la valeur N pour chaque spectre de travail en effectuant l'opération de sélection suivante :

-   on choisit dans le premier spectre de travail un nombre l de longueurs d'ondes consécutives égal à 735
-   on calcule des coefficients par décomposition en série de Fourier du premier spectre de travail,
-   on choisit les 20 premiers coefficients
-   on détermine un premier spectre de travail recomposé à partir des 20 premiers coefficients,
-   on calcule un paramètre STDm par la formule :

$$STDm = [\Sigma_i(A_i - A'_i)^2/l]^{\frac{1}{2}}$$

dans laquelle :

.   Ai représente la valeur en absorbance transformée mathématiquement pour la longueur d'onde i du premier spectre de travail,
.   A'i représente la valeur en absorbance transformée mathématiquement pour la longueur d'onde i du premier spectre de travail recomposé,
.   i varie de 1 à **735,**
.   l représente le nombre de longueurs d'ondes du spectre égal à 735

-   on réitére le calcul du paramètre STDm avec les 20+j premiers coefficients, j variant de 1 à 735/4,
-   on compare les valeurs STDm ainsi obtenues avec une valeur de seuil STDs,
-   on retient comme valeur de n le nombre de coefficients correspondant à la valeur STDm immédiatement supérieure à une valeur de seuil STDs égale à l'écart type de répétabilité connu du spectromètre proche infrarouge soit 0,18 x 10⁻³. On obtient ainsi n = 107.

On effectue les mêmes opérations avec le deuxième spectre de travail, ce qui donne une valeur n = 42.

On effectue cette opération de sélection pour chaque gamme de longueurs d'ondes.

On recompose par transformation de Fourier inverse le premier spectre de travail à partir des 107 coefficients retenus pour déterminer un premier spectre recomposé en appliquant la formule suivante :

$$y'_i = 1/N\, \Sigma_k\, F(k)\, \exp(+j2\pi ik/N)$$

Dans laquelle :

y'i représente les valeurs recomposées du spectre de travail

N représente le nombre de longueurs d'ondes égal à 735 pour le premier spectre de travail et 600 pour le deuxième spectre de travail,

k varie de 1 à 107 pour le premier spectre de travail

k varie de 1 à 42 pour le deuxième spectre de travail

F(k) représente les coefficients résultants de la décomposition en série de Fourier à la fréquence 1/N,

On calcule un premier indicateur de bon fonctionnement de l'unité de fabrication d'essence à partir des valeurs de chaque premier spectre de travail et des valeurs des spectres de travail recomposés, en appliquant la formule suivante :

$$STD = [\Sigma_j(A_j-A'_j)^2/I]^{\frac{1}{2}}$$

dans laquelle :

- STD représente le premier indicateur de bon fonctionnement de l'unité de fabrication d'essence,
- Aj représente la valeur en absorbance dérivée pour la longueur d'onde j de chaque premier spectre de travail,
- A'j représente la valeur en absorbance dérivée pour lalongueur d'onde j de chaque premier spectre de travail recomposé avec les 107 coefficients de Fourier choisis,
- l représente le nombre de longueurs d'ondes choisi égal à 735.
- j varie de 1 à l,

On calcule un deuxième indicateur de bon fonctionnement de l'unité de fabrication d'essence à partir des valeurs de chaque deuxième spectre de travail et des valeurs des spectres de travail recomposés selon le même procédé, la valeur de j variant de 1 à l et la valeur de l étant égale à 600.

On obtient ainsi une suite de valeurs de ces deux indicateurs à chacun des instants auxquels la mesure d'absorbance de l'essence fabriquée est réalisée.

Si les valeurs de ces indicateurs évoluent au delà de limites jugées acceptables ou de manière non aléatoire l'opérateur de conduite de l'unité de fabrication concernée est alerté de l'apparition d'un dysfonctionnement de son unité.

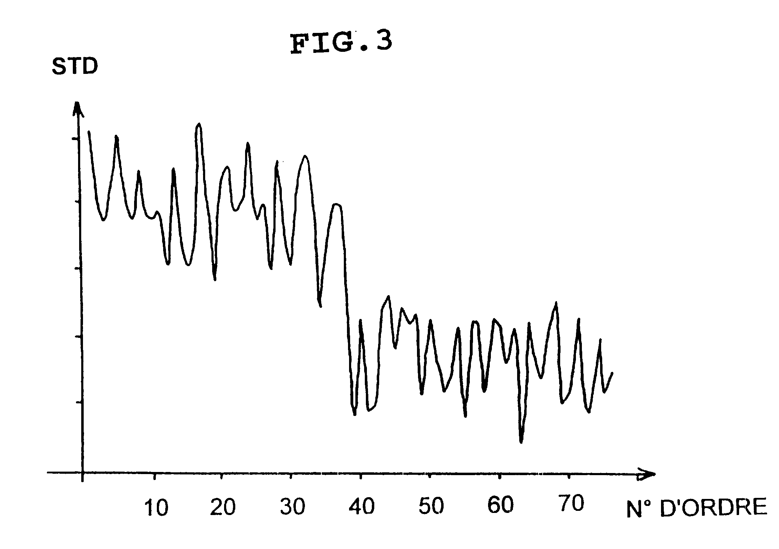

A titre d'exemple on trouvera représentée sur la figure 3 la valeur de l'indicateur de fonctionnement de l'unité de production d'essence, à chacun des instants auxquels la mesure d'absorbance de l'essence produite est réalisée, repéré par un numéro d'ordre, pour des spectres de travail comprenant chacuns 735 longueurs d'ondes comprises entre 1 090 et 1 640 nm uniformément réparties au pas de 0,75 nm, avec un nombre de coefficients de Fourier égal à 107. On constate que la valeur moyenne de cet indicateur chute brusquement entre les abscisses 35 et 38. Cette variation est caractéristique d'un dysfonctionnement dont l'origine peut être recherchée par des méthodes conventionnelles d'analyse de problèmes.

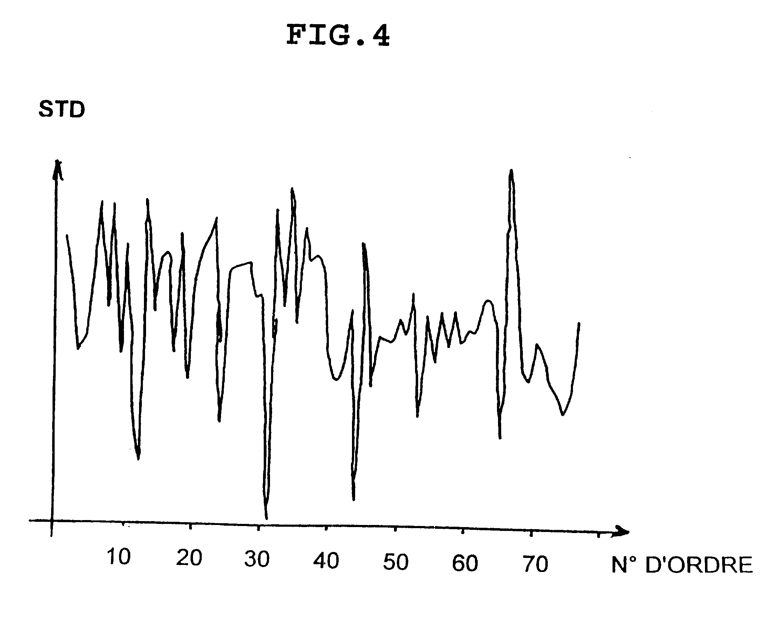

Sur la figure 4. on a reproduit l'évolution dans le temps de l'indicateur de fonctionnement de l'unité calculé avec des spectres de travail comportant 600 longueurs d'ondes uniformément réparties sur la plage 1 090 à 1 540 nm au pas de 0,75 nm. On constate une évolution cyclique caractéristique de recalages périodiques de l'unité de fabrication.

Chaque spectre peut avoir plusieurs caractéristiques STD qui correspondent à des gammes de longueurs d'ondes différentes. Ainsi en cas de disfonctionnement le procédé de l'invention permet de signaler la bande de longueur d'ondes incriminée.

Grâce à la visualisation des indicateurs de bon fonctionnement, il est possible de détecter très tôt un dysfonctionnement, de corriger le problème et ainsi d'éviter que les caractéristiques du produit fabriqué sortent des limites de spécifications.

Un autre avantage de l'invention est de permettre l'enrichissement de la base d'apprentissage utilisée pour établir le modèle, de telle sorte que cette base soit homogène d'une production, évolutive et dépourvue de spectres liés à des problèmes de fonctionnement.

Dans les exemples ci-dessus le spectromètre est réputé en bon état de fonctionnement.

Un autre but du procédé de l'invention est de détecter les défauts de fonctionnement du spectromètre proche infrarouge. L'unité de fabrication étant réputée correctement réglée, les variations des indicateurs de bon fonctionnement calculés selon le procédé de l'invention sont représentatifs des dysfonctionnements du spectromètre.

**Revendications**

1.  Procédé de suivi et de surveillance du fonctionnement d'une unité de fabrication d'un produit et/ou d'un spectromètre proche infrarouge alimenté par ledit produit, ledit spectromètre délivrant des spectres constitués par des séries de valeurs d'absorbance pour différentes valeurs de longueurs d'ondes, consistant à exécuter les étapes suivantes :

    - enregistrer périodiquement sous forme de données numériques les spectres issus du spectromètre proche infrarouge,
    - transformer mathématiquement les données numériques de chaque spectre enregistré, pour obtenir des spectres transformés,
    - constituer une suite de spectres de travail à partir des valeurs précédemment obtenues, en sélectionnant dans chaque spectre transformé, un ensemble de longueurs d'ondes consécutives caractéristiques du produit fabriqué,

    caractérisé en ce qu'il consiste à exécuter en plus les étapes suivantes :

    - calculer des coefficients par décomposition en série de chaque spectre de travail,
    - choisir un nombre n limité de coefficients en effectuant une opération de sélection,

- déterminer un spectre recomposé par recomposition de chaque spectre de travail à partir des coefficients choisis,
- calculer au moins un indicateur de bon fonctionnement de l'unité de fabrication et/ou du spectromètre proche infrarouge, au moyen d'une expression mathématique combinant les valeurs de chaque spectre de travail et celles du spectre recomposé correspondant,
- suivre dans le temps l'évolution de l'indicateur de bon fonctionnement.

2. Procédé selon la revendication 1 caractérisé en ce que la décomposition des spectres de travail est effectuée au moyen d'une décomposition en série choisie parmi les décompositions en série de Fourier, d'Hadamard et en série d'ondelettes.

3. Procédé selon la revendication 1 ou 2 caractérisé en ce que, la suite de spectres de travail comportant un premier spectre, l'opération de sélection consiste à déterminer le nombre n de coefficients en exécutant les étapes suivantes :

- choisir dans le premier spectre de travail un nombre I de longueurs d'ondes consécutives,
- calculer des coefficients par décomposition en série du premier spectre,
- choisir les p premiers coefficients,
- déterminer un premier spectre recomposé par recomposition du premier spectre de travail à partir des p premiers coefficients,
- calculer un paramètre STDm par la formule :

$$STDm = [\Sigma_i(A_i\text{-}A'_i)^2/I]^{\frac{1}{2}}$$

dans laquelle :

- Ai représente la valeur en absorbance transformée mathématiquement pour la longueur d'onde i du premier spectre de travail,
- A'i représente la valeur en absorbance transformée mathématiquement pour la longueur d'onde i du premier spectre de travail recomposé,
- i varie de 1 à I,
- I représente le nombre de longueurs d'ondes choisi
- réitérer le calcul du paramètre STDm avec les p+q premiers coefficients, q allant de 1 à ¼,
- comparer les valeurs STDm ainsi obtenues avec une valeur de seuil STDs,
- retenir le nombre n de coefficients correspondant à la valeur STDm immédiatement supérieure à la valeur STDs.

4. Procédé selon l'une des revendications 1,2 ou 3 caractérisé en ce que le spectromètre proche infrarouge ayant une répétabilité connue, la valeur de seuil STDs est choisie égale à l'écart type de ladite répétabilité.

5. Procédé selon l'une des revendications 1 à 4, caractérisé en ce que l'expression mathématique combinant les valeurs du spectre transformé et celles du spectre recomposé est la suivante :

$$STD = [\Sigma_j(A_j\text{-}A'_j)^2/I]^{\frac{1}{2}}$$

dans laquelle :

- STD représente l'indicateur de bon fonctionnement,
- Aj représente la valeur en absorbance transformée mathématiquement pour la longueur d'onde j de chaque spectre de travail,
- A'j représente la valeur en absorbance transformée mathématiquement pour la longueur d'onde j de chaque spectre de travail recomposé avec le nombre n de coefficients retenu,
- j varie de 1 à I
- I représente le nombre de longueurs d'ondes choisi

6. Procédé selon l'une des revendications 1 à 5 caractérisé en ce que pour suivre l'évolution dans le temps de l'indicateur de bon fonctionnement, on utilise une carte de contrôle monovariée.

7. Procédé selon l'une des revendications 1 à 6 caractérisé en ce que pour suivre l'évolution dans le temps de l'indicateur de bon fonctionnement, on utilise un diagramme causes/effets.

FIG.1

ABSORBANCE
DERIVEE

1640          1090          LONGUEURS
                            D'ONDES EN nm

EP 0 838 676 A1

ABSORBANCE DERIVEE

FIG.2

1640      1090    LONGUEURS D'ONDES EN nm

EP 0 838 676 A1

FIG.3

STD

N° D'ORDRE

EP 0 838 676 A1

FIG.4

Office européen
des brevets

**RAPPORT DE RECHERCHE EUROPEENNE**

Numéro de la demande

EP 97 40 2492

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP 0 706 041 A (BP CHEMICALS SNC) 10 avril 1996 <br> * page 2, ligne 33 – page 3, ligne 4 * | 1 | G01N21/35 <br> G01N33/28 |
| A | US 4 963 745 A (MAGGARD STEVEN M) 16 octobre 1990 <br> * colonne 1, ligne 56 – colonne 2, ligne 5 * | 1 | |
| A | EP 0 304 232 A (BP OIL INT) 22 février 1989 <br> * page 2, ligne 17 – page 3, ligne 57 * <br> * revendications 1,7,8 * | 1,2 | |

|  | DOMAINES TECHNIQUES RECHERCHES (Int.Cl.6) |
|---|---|
|  | G01N |

Le present rapport a ete etabli pour toutes les revendications

| Lieu de la recherche | Date d'achèvement de la recherche | Examinateur |
|---|---|---|
| LA HAYE | 28 janvier 1998 | Krametz, E |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-ecrite
P : document intercalaire

T : theorie ou principe à la base de l'invention
E : document de brevet antérieur, mais publié à la
   date de dépôt ou apres cette date
D : cite dans la demande
L : cite pour d'autres raisons

& : membre de la même famille, document correspondant

EPO FORM 1503 03 82 (P04C02)